# FASCICULE DE BREVET EUROPEEN

(11) **EP 0 634 660 B1**
(45) Date de publication et mention de la délivrance du brevet: **13.01.1999**
(21) Numéro de dépôt: 94401604.7
(22) Date de dépôt: 12.07.1994
(51) Int. Cl.: G01N 35/10, G01N 1/00

(54) **Dispositif de nettoyage d'un aiguille de prélèvement d'un liquide dans un flacon fermé**
Reinigungsvorrichtung für eine Kanüle zur Entnahme eine Flüssigkeitsprobe aus einer geschlossenen Flasche
Device for cleaning a needle for sampling liquid from a closed bottle

(30) Priorité: 15.07.1993 FR 9308671
(43) Date de publication de la demande: 18.01.1995
(73) Titulaire: ABX , Société Anonyme dite, F-34184 Montpellier Cédex 4 (FR)
(72) Inventeur: Le Comte, Roger, F-34280 Carnon (FR); Couderc, Guilhem, F-34430 St Jean de Vedas (FR); Champseix, Henri, F-34980 Montpellier sur Lez (FR)
(74) Mandataire: Lepeudry-Gautherat, Thérèse

(56) Documents cités:
- US-A- 3 719 086
- US-A- 3 748 911
- US-A- 3 991 627
- US-A- 4 624 148

## Description

L'invention a pour objet un dispositif de nettoyage d'une aiguille de prélèvement d'un liquide dans un flaconf fermé, et plus particulièrement de prélèvement de sang dans un appareil d'analyse hématologique et l'invention concerne essentiellement un mécanisme de nettoyage lié à un organe de guidage de l'aiguille de prélèvement.

Il existe des appareils automatiques d'analyse hématologique qui permettent, à partir d'un échantillon de sang, de déterminer des paramètres tels que le nombre de leucocytes et d'hématies, la quantité d'hémogolobine etc... Les échantillons de sang doivent pour cela être prélevés dans un flacon puis transférés dans un ou plusieurs petits récipients de l'appareil où ils sont soumis à des mesures appropriées. Pour prélever le sang dans un flacon fermé par un bouchon, on connaît la solution qui consiste à percer ledit bouchon à l'aide d'une aiguille qui plonge alors dans le liquide, et à aspirer la quantité de sang voulu au travers de ladite aiguille comme indiqué par exemple dans le US-3 991 627. Cette opération s'effectue aussi bien sur des flacons dont le bouchon de fermeture est en position supérieure ou sur des flacons renversés dont le bouchon est à la partie inférieure. Bien entendu des mécanismes appropriés tels que décrits dans le FR-2 120 300 et US-4 624 148 assurent - soit le déplacement de l'aiguille soit celui du flacon - pour assurer le perçage. Pour pouvoir prélever une quantité très précise de sang et la distribuer ensuite vers la ou les unités de mesure, il faut utiliser dans tous les cas une vanne d'échantillonnage montée sur le tuyau de prélèvement, entre l'aiguille et les analyseurs proprement dits. Cette vanne d'échantillonnage - surtout pour les quantités très faibles de sang que l'on veut prélever - constitue une pièce qui doit être minutieusement réalisée, qui est donc coûteuse et de réglage difficile. Et puis elle nécessite de subir un rinçage entre chaque prélèvement, en même temps que s'effectue le rinçage de l'aiguille. Celui-ci d'ailleurs est souvent assuré par le passage de l'aiguille mobile dans un boîtier de rinçage fixe montré par exemple dans le US-3 719 086, ou encore par un boîtier de rinçage coulissant sur une aiguille fixe de prélèvement comme le décrit le FR-A-2 606 885 au nom de la Demanderesse. La vanne d'échantillonnage évoquée plus haut est une nécessité sur les appareils existants dans la mesure où, dans les flacons d'échantillon de sang, il y a souvent une petite surpression ou une petite dépression d'air à l'intérieur, par rapport à l'air ambiant. Si l'on veut éviter l'usage de la vanne d'échantillonnage, et pouvoir cependant prélever exactement la quantité de sang prévue, il faudrait que les flacons à prélever soient toujours dans des conditions constantes de pression ce qui est loin d'être le cas.

La Demanderesse a trouvé une solution à ce problème qui lui permet de s'affranchir de l'usage de cette vanne d'échantillonnage et qui en même temps facilite l'opération de rinçage de l'aiguille de prélèvement.

Un objet principal de la présente invention consiste par conséquent en un dispositif de nettoyage d'une aiguille de prélèvement d'un liquide dans un flacon fermé par un bouchon dans lequel ladite aiguille est montée sur un mécanisme mobile se déplaçant de haut en bas pour assurer le prélèvement du liquide dans le flacon et dans lequel une aiguille de perçage du bouchon est traversé par l'aiguille de prélèvement, dispositif selon lequel l'aiguille de prélèvement traverse un organe de guidage et l'organe de guidage est monté à la partie inférieure d'une platine-support intégrée dans l'appareil de prélèvement, ledit organe de guidage servant aussi de support à l'aiguille de perçage et disposant d'au moins un conduit communiquant avec l'aiguille de perçage pour la mise à l'air libre du flacon et le rinçage de l'aiguille de prélèvement.

Selon une caractéristique particulière de l'invention l'organe de guidage de l'aiguille de prélèvement est une tête de percussion fixée à la base de la platine servant de support au dispositif, et perçée d'un puits vertical qui la traverse entièrement et à l'intérieur duquel est fixée l'aiguille de perçage.

Selon une variante préférentielle de l'invention, l'organe de guidage de l'aiguille de prélèvement est une cuve de dilution fixée à la base de la platine servant de support au dispositif, l'aiguille de perçage étant maintenue à l'intérieur d'un bouchon rapporté au-dessous de la cuve, le fond de cette dernière communiquant avec l'aiguille de perçage par un trou vertical de guidage. Avantageusement, des orifices latéraux mettent en communication avec l'extérieur la chambre interne de la cuve de dilution.

D'autres caractéristiques et avantages de l'invention ressortiront de la description qui va suivre d'exemples de réalisation de l'invention dans lesquels il est fait référence aux dessins annexés qui représetent.

Figure 1 une vue en coupe verticale d'une première variante de réalisation du dispositif.

Figure 2 une vue en coupe à plus grande échelle de l'aiguille de perçage et de l'aiguille de prélèvement.

Figures 3 et 4 des coupes verticales respectivement de face et de profil d'une autre variante de réalisation du dispositif.

Figures 5 et 6 des coupes verticales à plus grande échelle, respectivement de face et de profil, de la base de la cuve de dilution.

Figures 7 et 8 des coupes verticales, respectivement de face et de profil d'une autre variante de cuve de dilution.

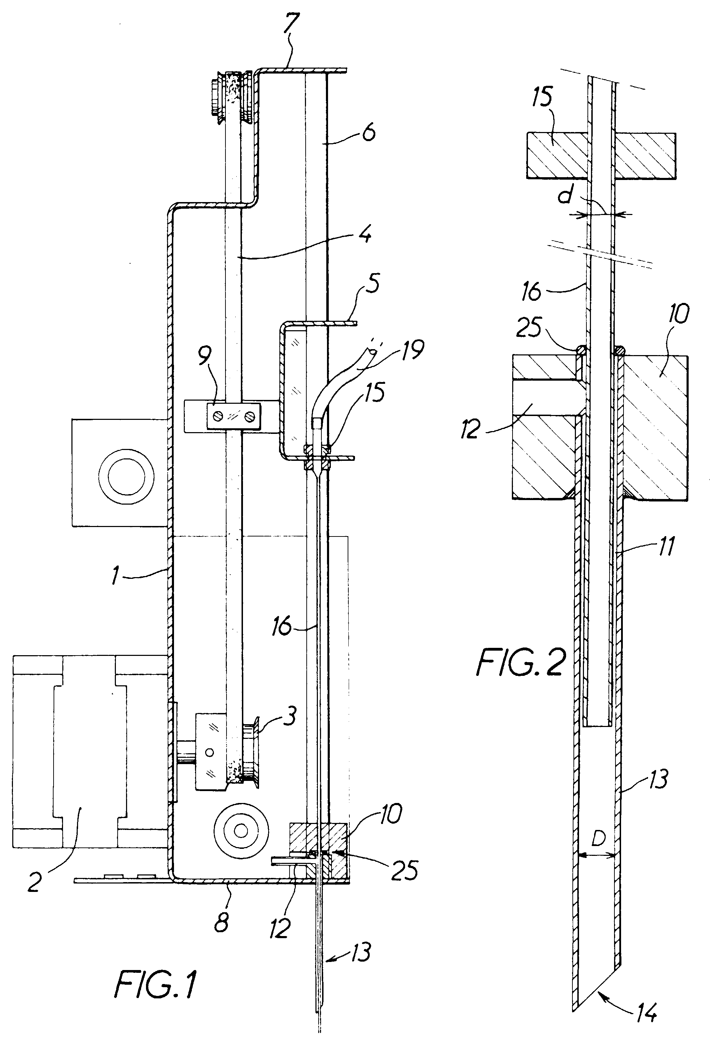

On a représenté à la figure 1 la platine 1 servant de support au dispositif, destinée à s'intégrer à l'avant d'un appareil de prélèvement et d'analyse. La platine 1 est susceptible de se déplacer latéralement dans l'appareil au-dessus de cuves de dilution, de rinçage ou de comptage non représentées. Cette platine a la forme générale d'une coquille plus haute que large et ouverte vers l'extérieur, dont la paroi constituant le fond sert de support à un moteur 2 qui entraîne en rotation un pignon cranté 3 à axe horizontal. Par l'intermédiaire d'une courroie sans fin 4 faisant retour sur une poulie de renvoi, - courroie s'étendant sur toute la hauteur de la platine -, le moteur assure le déplacement d'un coulisseau 5 le long de deux colonnes de guidage 6 qui se développent verticalement entre un rebord horizontal supérieur 7 et un rebord horizontal inférieur 8 de la platine 1. Pour cela la partie arrière du coulisseau porte un clips de serrage 9 qui pince un des brins verticaux de la courroie 4. Sur le rebord inférieur 8 de la platine est rapportée une tête de percussion 10 qui est donc une pièce fixe par rapport à l'appareil. Ladite tête de percussion mieux visible sur la figure 2 se présente comme un petit boîtier percé en son centre d'un puits vertical 11 qui le traverse entièrement, lequel puits communique également avec l'extérieur par un petit conduit horizontal 12 creusé dans la tête de percussion. A l'intérieur du puits est fixée une aiguille de perçage 13 de diamètre intérieur D qui s'étend vers le bas au-dessous de la platine 1. Un orifice latéral est prévu sur l'aiguille de perçage, au droit du conduit 12 pour faire communiquer ce dernier avec l'intérieur de l'aiguille. L'extrémité inférieure de celle-ci est ouverte, comme représenté à la figure 2, par un orifice biseauté 14. En variante d'autres profils d'embouts pourraient être prévus, notamment un orifice droit.

Sur le coulisseau 5 est fixée une pièce d'accrochage 15 d'une aiguille de prélèvement 16 ayant un diamètre extérieur d légèrement plus faible que le diamètre intérieur D de l'aiguille de perçage 13. L'aiguille de prélèvement 16 se développe verticalement sous le coulisseau, sur une longueur approximativement égale à la hauteur de la platine et coaxialement à l'aiguille de perçage. L'ouverture d'extrémité de l'aiguille de prélèvement est cylindrique. L'étanchéité entre l'aiguille de prélèvement 16 et l'aiguille de perçage 13 est assurée par un joint torique 25 monté au sommet de la tête de percussion 10.

A la figure 1 le coulisseau 5 a été représenté sensiblement à mi-hauteur de sa course. L'extrémité 17 de l'aiguille de prélèvement 16 déborde alors légèrement de l'orifice biseauté 14 de l'aiguille de perçage 13. On comprend qu'en position haute du coulisseau, l'aiguille de prélèvement s'efface à l'intérieur de l'aiguille de perçage, sa pointe se trouvant au niveau du sommet de la tête de percussion. En position basse au contraire, l'aiguille de prélèvement déborde nettement au-dessous de l'extrémité de l'aiguille de perçage. La différence entre les diamètres D et d des deux aiguilles autorise un coulissement aisé de l'une dans l'autre avec un faible jeu. La tête de percussion 10 constitue un organe de guidage pour l'aiguille de prélèvement. Le sommet ouvert de l'aiguille de prélèvement 16 est relié par une tubulure flexible 19 à un récipient d'analyse de l'appareil.

Au début de l'opération de prélèvement la platine 1 supportant la tête de percussion 10 et le coulisseau 5 est positionnée au-dessus d'un flacon d'échantillon disposé dans un réceptacle non représenté, bouchon vers le haut, sous l'aiguille fixe de perçage 13. Le coulisseau 5 est alors en position haute. Le flacon, mû par un mécanisme mobile approprié se déplace de bas en haut et son bouchon se perce sur l'aiguille de perçage, sans que l'orifice de cette dernière ne plonge dans le liquide du flacon. En variante, le flacon pourrait être fixe et toute la platine 1 pourrait descendre vers le flacon pour percer son bouchon. Dès le perçage, l'air, contenu dans le flacon peut communiquer avec l'extérieur par l'aiguille 13 et le conduit 12. Cette mise à l'air libre élimine la surpression ou la dépression d'air pouvant exister dans le flacon. Le prélèvement peut alors être assuré. Le moteur 2 fait descendre le coulisseau 5 à l'aide de la courroie crantée 4 ; l'aiguille de prélèvement 16 descend à l'intérieur de l'aiguille fixe de perçage 13 jusqu'à plonger au fond du flacon. Puis on aspire le sang par l'extrémité de l'aiguille. Etant dans des conditions constantes de pression, on prélève de façon précise la quantité de sang voulue qui s'immobilise dans l'aiguille. Le moteur commande ensuite la remontée de l'aiguille. Puis le mécanisme mobile déplace de haut en bas le flaçon pour assurer la déperforation de son bouchon. La platine 1 se déplace ensuite latéralement dans l'appareil au-dessus de cuves de dilution, de rinçage ou de comptage. L'ensemble de percussion est donc fixe par rapport au tube de sang pendant l'opération de perçage, mais est déplacé durant la suite des opérations.

Au cours de cette phase de déplacement, la platine 1 se positionne au-dessus d'une cuve de dilution. Ensuite la distribution d'un liquide se fait par le conduit 12 ou avantageusement par un second conduit débouchant également dans l'aiguille de perçage 13, afin de rincer le sang au niveau du joint torique 25 et le long de la paroi intérieure de l'aiguille 13. Ce sang résiduel ne fait pas partie de la quantité dosée et doit donc être éliminé avant la première opération de dilution. Il y a ensuite une distribution de diluant à l'intérieur de l'aiguille de prélèvement pour chasser le contenu de sang dosé hors de l'aiguille et le mélanger à une quantité dosée de diluant afin d'effectuer une dilution selon un taux connu.

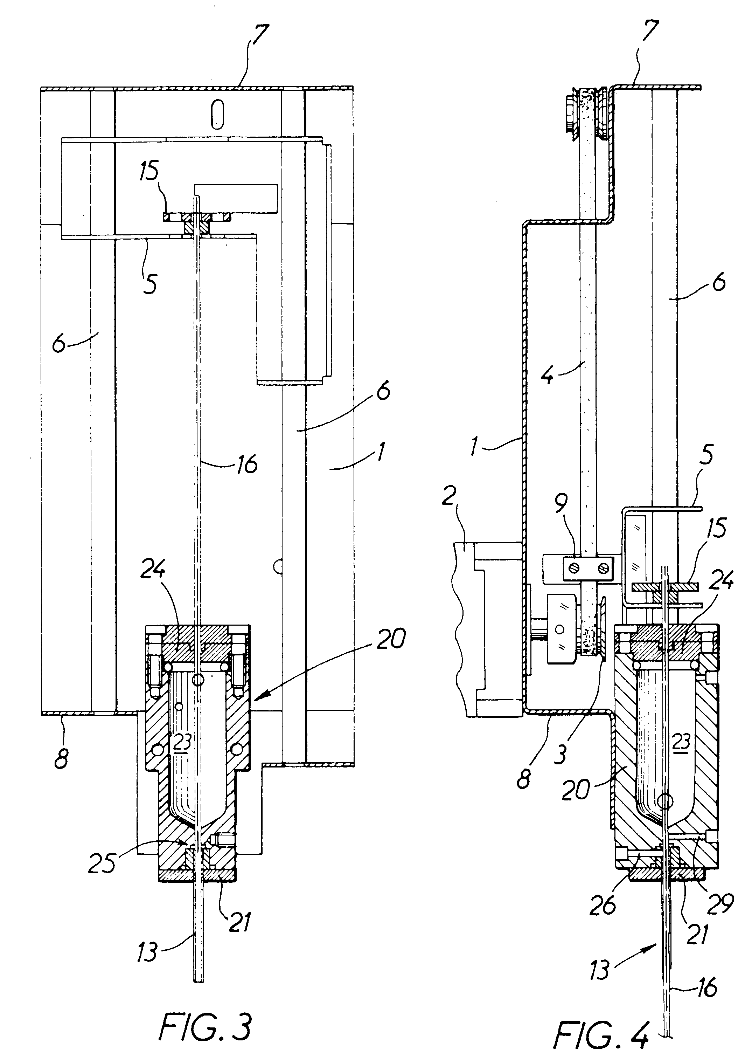

On décrit maintenant une autre variante de réalisation de l'invention illustrée aux figures 3 à 6, dans lesquelles on retrouve avec les mêmes références les éléments décrits précédemment.

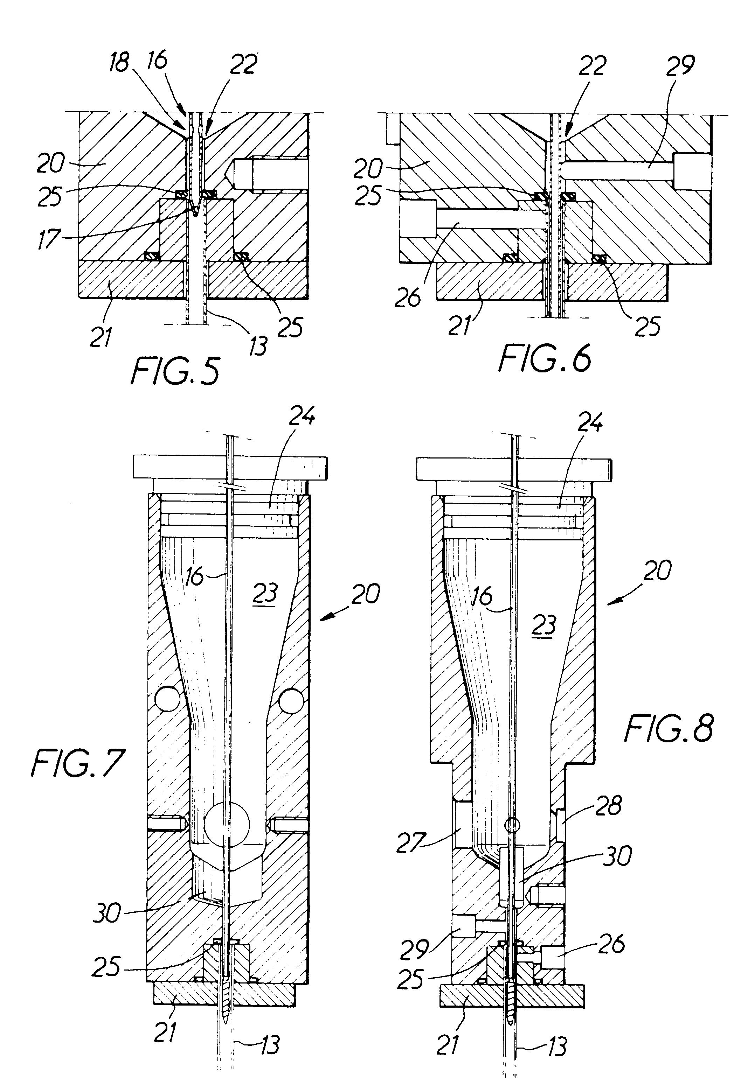

Cette variante se différencie de la première essentiellement par le fait que la tête de percussion est remplacée par une cuve de dilution désignée dans son ensemble par la référence 20. Cette cuve est également fixée sur le rebord inférieur 8 de la platine 1. L'aiguille de perçage 13 est maintenue à l'intérieur d'un bouchon 21 rapporté au-dessous de la cuve. Elle communique par un trou vertical de guidage 22 prévu dans la base de la cuve, avec la chambre intérieure 23, dont le fond est de profil conique. A sa partie supérieure la cuve est fermée par un bouchon 24. Des joints d'étanchéité 25 sont prévus entre la cuve et le bouchon inférieur 21, notamment au sommet de l'aiguille de perçage pour assurer l'étanchéité entre celle-ci et l'aiguille de prélèvement. On notera que dans cette variante, l'aiguille de prélèvement 16 diffère de la précédente par le fait que son extrémité inférieure se termine par une pointe fermée 17, mais dispose un peu plus haut de deux petits orifices latéraux 18, comme on peut le voir plus précisément sur la figure 5. D'autre part le trou de guidage 22 communique avec l'extérieur par une ouverture 29 creusée dans le corps de la cuve, ouverture qui débouche sensiblement à mi-hauteur du trou et qui peut se raccorder à un conduit de vidange de cuve. Un peu plus bas, dans le bouchon et l'aiguille de perçage 13, débouche une autre ouverture 26 de mise à l'air libre ou de rinçage.

Après le perçage du flacon par l'aiguille de perçage 13, sa mise à l'air libre est assurée par l'ouverture 26 en communication avec l'extérieur. Ensuite, comme dans le cas précédent, le moteur 2 assure la descente de l'aiguille de prélèvement 16 dont l'extrémité 17 était immobilisée en haut du bouchon 21 comme on le voit plus précisément à la figure 5. L'aiguille coaxiale à la chambre 21 descend par le trou vertical 22 puis à l'intérieur par l'aiguille de perçage 13 avant de plonger au fond du flacon. La petite quantité voulue de sang est aspirée dans l'aiguille 16 puis cette dernière remonte dans une position haute, pour laquelle les orifices latéraux 18 de l'aiguille (visibles figure 5) se trouvent immobilisés à la base de la chambre 23. De même, comme pour la première variante, le mécanisme mobile déplace de haut en bas le flacon pour assurer la déperforation de son bouchon. Puis la platine 1 se positionne au-dessus d'une cuve de rinçage, ou encore une cuve de rinçage se place sous la platine, évitant ainsi sa translation latérale. La distribution de liquide se fait par le conduit 26 ou avantageusement par un second conduit débouchant dans l'aiguille de perçage 13. Par le conduit de raccordement au sommet de l'aiguille, on injecte alors un diluant, en sens inverse de l'aspiration précédente. La poussée du diluant a pour effet de faire refluer le sang retenu dans l'aiguille vers la cuve 20 par lesdits orifices latéraux 18. La dilution s'effectue donc dans la cuve. Puis elle est évacuée par l'ouverture 29, grâce à la pression dans la cuve ou la dépression appliquée à cette ouverture. On effectue le rinçage de l'aiguille 13 par injection de liquide dans l'ouverture 26. De son côté, l'aiguille de prélèvement 16 est nettoyée extérieurement par frottement sur un joint d'étanchéité 25 tandis que l'intérieur a été nettoyé par le diluant.

Un des avantages d'une telle cuve de dilution est d'être réalisée en une seule pièce et, de servir d'organe de guidage à l'aiguille en même temps que d'assurer son nettoyage.

Les figures 7 et 8 illustrent une autre variante de réalisation d'une telle cuve de dilution, qui se différencie de la précédente notamment par le profil de la chambre intérieure 23. Sur cette figure, les mêmes pièces que celles de la variante des figures 3 et 4 portent les mêmes références. On notera que le fond de la cuve en V est creusé pour former une alvéole plate 30 que traverse en son centre l'aiguille de prélèvement 16 avant d'atteindre le trou de guidage. Cette alvéole est destinée à la mesure de l'hémoglobine par densité optique. On notera que la cuve de dilution dispose avantageusement d'au moins un système de mesure spectrophotométrique.

Sur le côté de la chambre 23, sont prévus des orifices latéraux 27 et 28, servant à positionner un organe de comptage, par exemple un dispositif électronique à rubis et électrode.

## Revendications

1. Dispositif de nettoyage d'une aiguille de prélèvement (16) d'un liquide dans un flacon fermé par un bouchon dans lequel ladite aiguille de prélèvement est montée sur un mécanisme mobile (5) se déplaçant de haut en bas pour assurer le prélèvement de liquide dans le flacon et dans lequel une aiguille (13) de perçage du bouchon est traversée par l'aiguille de prélèvement, caractérisé en ce que l'aiguille de prélèvement traverse un organe de guidage (10,20) et l'organe de guidage (10, 20) est monté à la partie inférieure d'une platine-support (1) intégrée dans l'appareil de prélèvement, et en ce que ledit organe de guidage sert aussi de support à l'aiguille de perçage (13) et dispose d'au moins un conduit (12, 26, 29) communiquant avec l'aiguille de perçage pour la mise à l'air libre du flacon et le rinçage de l'aiguille de prélèvement (16).

2. Dispositif de nettoyage selon la revendication 1, caractérisé en ce que l'organe de guidage de l'aiguille de prélèvement (16) est une tête de percussion (10) fixée à la base de la platine (1) servant de support au dispositif, et percée d'un puits vertical (11) qui la traverse entièrement et à l'intérieur duquel est fixée l'aiguille de perçage (13).

3. Dispositif de nettoyage selon la revendication 1, caractérisé en ce que l'organe de guidage de l'aiguille de prélèvement (16) est une cuve de dilution (20) fixée à la base de la platine (1) servant de support au dispositif, en ce que l'aiguille de perçage (13) est maintenue à l'intérieur d'un bouchon (21) rapporté au-dessous de la cuve, le fond de cette dernière communiquant avec l'aiguille de perçage par un trou vertical de guidage (22).

4. Dispositif de nettoyage selon la revendication 3, caractérisé en ce que des orifices latéraux (27, 28) mettent en communication avec l'extérieur la chambre interne (23) de la cuve de dilution (20).

5. Dispositif de nettoyage selon la revendication 4, caractérisé en ce que sur les orifices latéraux (27, 28) est positionné un organe de comptage.

6. Dispositif de nettoyage selon la revendication 2, caractérisé en ce qu'au moins un joint d'étanchéité (25) est monté au sommet de la tête de percussion (10) pour assurer l'étanchéité entre l'aiguille de prélèvement (16) et l'aiguille de perçage (13).

7. Dispositif de nettoyage selon la revendication 3, caractérisé en ce que des joints d'étanchéité (25) sont prévus entre la cuve de dilution (20) et le bouchon (21), au sommet de l'aiguille de perçage (13).

8. Dispositif de nettoyage selon les revendications 1 et 4, caractérisé en ce que le fond de la chambre interne (23) de la cuve de dilution (20) est de profil conique.

9. Dispositif de nettoyage selon les revendications 1 et 7, caractérisé en ce que le fond de la chambre interne (23) de la cuve de dilution (20) est creusé pour former une alvéole plate (30) que traverse en son centre l'aiguille de prélèvement (16).

10. Dispositif de nettoyage selon la revendication 3, caractérisé en ce que la cuve de dilution dispose d'au moins un système de mesure spectrophotométrique.

## Patentansprüche

1. Reinigungsvorrichtung für eine Kanüle (16) zur Entnahme einer Flüssigkeit aus einem geschlossenen Kolben durch einen Stopfen, wobei die Entnahmekanüle an einem beweglichen Mechanismus (5) angebracht ist, der sich von oben nach unten bewegt, um die Entnahme der Flüssigkeit aus dem Kolben sicherzustellen, und wobei eine Kanüle (13) zum Durchstechen des Stopfens von der Entnahmekanüle durchquert wird, dadurch gekennzeichnet, daß die Entnahmekanüle ein Führungsglied (10, 20) durchquert und das Führungsglied (10, 20) am unteren Teil eines in die Entnahmeeinrichtung integrierten Stützträgers (1) angebracht ist und daß das Führungsglied auch als Stütze für die Durchstechkanüle (13) dient und mindestens eine Leitung (12, 26, 29) aufweist, die mit der Durchstechkanüle in Verbindung steht, um den Kolben mit der Atmosphäre zu verbinden und die Entnahmekanüle (16) zu spülen.

2. Reinigungsvorrichtung nach Anspruch 1, dadurch gekennzeichnet, daß das Glied zur Führung der Entnahmekanüle (16) ein Schlagkopf (10) ist, der an der Basis des als Stütze für die Vorrichtung dienenden Trägers (1) befestigt ist und durch den eine Vertikalbohrung (11) verläuft, die ihn ganz durchquert und in der die Durchstechkanüle (13) befestigt ist.

3. Reinigungsvorrichtung nach Anspruch 1, dadurch gekennzeichnet, daß das Glied zur Führung der Entnahmekanüle (16) ein Verdünnungsgefäß (20) ist, das an der Basis des als Stütze für die Vorrichtung dienenden Trägers (1) befestigt ist, und daß die Durchstechkanüle (13) im Innern eines unter dem Gefäß angebrachten Stopfens (21) gehalten wird, wobei der Boden des Gefäßes durch ein Vertikalführungsloch (22) mit der Durchstechkanüle in Verbindung steht.

4. Reinigungsvorrichtung nach Anspruch 3, dadurch gekennzeichnet, daß seitliche Öffnungen (27, 28) die Innenkammer (23) Verdünnungsgefäßes (20) mit der Umgebung verbinden.

5. Reinigungsvorrichtung nach Anspruch 4, dadurch gekennzeichnet, daß ein Zählglied an den seitlichen Öffnungen (27, 28) positioniert ist.

6. Reinigungsvorrichtung nach Anspruch 2, dadurch gekennzeichnet, daß mindestens eine Dichtung (25) am oberen Ende des Schlagkopfes (10) angebracht ist, um die Dichtigkeit zwischen der Entnahmekanüle (16) und der Durchstechkanüle (13) zu gewährleisten.

7. Reinigungsvorrichtung nach Anspruch 3, dadurch gekennzeichnet, daß Dichtungen (25) zwischen dem Verdünnungsgefäß (20) und dem Stopfen (21) am oberen Ende der Durchstechnadel (13) vorgesehen sind.

8. Reinigungsvorrichtung nach den Ansprüchen 1 und 4, dadurch gekennzeichnet, daß der Boden der Innenkammer (23) des Verdünnungsgefäßes (20) ein konisches Profil aufweist.

9. Reinigungsvorrichtung nach den Ansprüchen 1 und 7, dadurch gekennzeichnet, daß der Boden der Innenkammer (23) des Verdünnungsgefäßes (20) vertieft ist und so eine flache Zelle (30) bildet, deren Mitte von der Entnahmekanüle (16) durchquert wird.

10. Reinigungsvorrichtung nach Anspruch 3, dadurch gekennzeichnet, daß das Verdünnungsgefäß mindestens ein spektralphotometrisches Meßsystem aufweist.

## Claims

1. Device for cleaning a needle (16) for sampling a liquid from a bottle closed by a bung, wherein the said sampling needle is mounted on a mobile mechanism (5) that moves downwards to ensure the sampling of liquid from the bottle and wherein the sampling needle passes through a needle (13) for piercing the bung, characterized in that the sampling needle passes through a guide member (10, 20) and the guide member (10, 20) is mounted on the lower portion of a support bracket (1) integrated in the sampling apparatus, and in that the said guide member also serves to support the piercing needle (13) and is provided with at least one conduit (12, 26, 29) communicating with the piercing needle for venting the bottle to ambient air and rinsing the sampling needle (16).

2. Cleaning device according to claim 1, characterized in that the member for guiding the sampling needle (16) is a percussion head (10) fixed to the base of the bracket (1) serving as a support for the device, pierced by a vertical shaft (11) which passes through it completely and inside which is fixed the piercing needle (13).

3. Cleaning device according to claim 1, characterized in that the member for guiding the sampling needle (16) is a dilution vessel (20) fixed to the base of bracket (1) serving as a support for the device, in that the piercing needle (13) is held inside a plug (21) mounted under the vessel, the bottom of the latter communicating with the piercing needle via a vertical guide hole (22).

4. Cleaning device according to claim 3, characterized in that lateral orifices (27, 28) place the internal chamber (23) of the dilution vessel (20) in communication with the outside.

5. Cleaning device according to claim 4, characterized in that a metering member is positioned on the lateral orifices (27, 28).

6. Cleaning device according to claim 2, characterized in that at least one seal (25) is mounted on the top of the percussion head (10) to ensure tightness between the sampling needle (16) and the piercing needle (13).

7. Cleaning device according to claim 3, characterized in that seals (25) are provided between the dilution vessel (20) and the plug (21), at the top of the piercing needle (13).

8. Cleaning device according to claims 1 and 4, characterized in that the bottom of the inner chamber (23) of the dilution vessel (20) has a conical profile.

9. Cleaning device according to claims 1 and 7, characterized in that the bottom of the inner chamber (23) of the dilution vessel (20) is hollowed to form a flat alveole (30) through the centre of which passes the sampling needle (16).

10. Cleaning device according to claim 3, characterized in that the dilution vessel is provided with at least one spectrophotometric measuring system.
